# EUROPEAN PATENT APPLICATION

(11) **EP 0 614 883 A1**
(43) Date of publication of application: **14.09.1994**
(21) Application number: 94200563.8
(22) Date of filing: 05.03.1994
(51) Int. Cl.: C07C 273/04

(54) **Process for synthetizing urea from ammonia and carbon dioxide, with total carbon dioxide conversion**

(30) Priority: 10.03.1993 IT MI930455
(71) Applicant: SNAMPROGETTI S.p.A., I-20097 San Donato Milanese (Milano) (IT)
(72) Inventor: Rescalli, Carlo, I-20097 San Donato Milanese (Milan) (IT)
(74) Representative: Fusina, Gerolamo

(57) **Abstract**

A process for synthetizing urea by starting from NH₃ and CO₂, with total CO₂ conversion is disclosed which comprises:
(a) feeding, in countercurrent mode, to a reactive distillation column, NH₃ to the column bottom and CO₂ to the central column zone, with a liquid bottom stream being obtained which contains urea, NH₃ and water, and an overhead gas and/or supercritical stream constituted by NH₃ and water being obtained;
(b) feeding the liquid stream coming from the bottom of the reactive distillation column to one or more separation steps, with NH₃ containing streams and an urea containing stream to be sent to a subsequent prilling step being obtained;
(c) feeding the gas and/or supercritical stream coming from the reactive distillation column head, to a distillation column from the bottom of which water and from the head of which NH₃ are separated.

## Description

The present invention rotates to a process for synthetizing urea by starting from ammonia and carbon dioxide, with total conversion of CO₂ being obtained.

All industrial processes for preparing urea are based on the direct synthesis according to the reaction:

2NH₃ + CO₂ ⇄ CO(NH₂)₂ + H₂O (1)

Actually, this reaction takes place in two well distinct steps:

2NH₃ + CO₂ ⇄ (NH₂)COONH₃ (1a)

(NH₂)COONH₄ ⇄ CO(NH₂)₂ + H₂O (1b)

The first step is an exothermic equilibrium reaction having a high reaction rate already at room temperature, but a favourable equilibrium, at such high temperatures as required by reaction (1b), only under high pressures; the second reaction is endothermic, with a meaningful reaction rate only at high temperatures (higher than, or equal to, 150°C) and an equilibrium which, at 185°C, leads to a conversion of only approximately 53% of CO₂: this value can be suitably increased by increasing the ratio of NH₃/CO₂, but is considerably decreased in the presence of water. Water has a negative effect also on the kinetics of the reaction: its presence causes therefore particularly negative effects on the overall reaction.

According to the classic industrial processes, the synthesis is carried out inside a single-stage reactor fed in co-current mode, from the bottom, with NH₃, CO₂ and aqueous solution of recycled ammonium carbonate, at temperatures comprised within the range of from 170 to 200°C, under pressures comprised within the range of from 150 to 250 atm, with a molar ratio of NH₃/CO₂ in the feedstock comprised within the range of from 2.5 to 4.5. The product discharged from reactor head displays conversions of 60-65% of fed CO₂. Besides formed water and excess NH₃ used, the effluent stream from the reaction contains large amounts of CO₂ as non converted ammonium carbamate. The separation of urea from these products is carried out in a specific section of the facility, in which basically some flash treatments are carried out at high temperature and under decreasing pressures in order to decompose carbamate into NH₃ and CO₂ and simultaneously remove these products and any reaction water, with urea being made available for the subsequent prilling step.

The carbamate separation section displays low operation costs, thanks to the recovery of a large amount of heat from the unit, but, simultaneously, requires extremely high costs of investment which cause enormous increases in end product costs.

From this section of the facility, all CO₂ and a portion of NH₃, owing to their simultaneous presence, are made available for being recycled as ammonium salts (carbonate and/or hydrogencarbonate and/or carbamate, according to temperature), requiring water to be used as the solvent means for their transport: owing to the already indicated reasons, such a requirement causes decidedly negative effect on reactor operations.

In order to better clarify the above, we regard it useful to evidence that the water amount recycled to the reactor for the above said transport, is from a quantitative viewpoint, of the order of magnitude of water amounts produced during the course of the reaction.

It should be furthermore observed that the traditional single-step, co-current reactor is particularly penalized because, besides receving large amounts of water from the recycle lines already from the beginning, water concentration inside it is also increasing with progressing reaction: the highest water concentration is actually found precisely in the end reactor zone, in which, on the contrary, having a low water concentration would be much more useful in order to favour the equilibrium,as well as the kinetics, of reaction (1b).

In order to increase as far as possible the conversion of CO₂ in the traditional facilities, also the temperature and pressure conditions are forced: however, even so, conversion levels of 60 - 65% cannot be exceeded.

We have found now a process which makes it possible the total conversion of fed CO₂ to be achieved, with the difficulties which affect the industrial process being thus overcome.

The process according to the present invention for synthetizing urea from ammonia and carbon dioxide is characterized in that said process comprises the following operations:
(a) feeding the reactants, in countercurrent mode, to a reactive distillation column (column-reactor) kept under such temperature and pressure conditions, that in each step a liquid phase (constituted by ammonium carbamate and/or urea and ammonia and/or water) and a gas and/or supercritical phase (constituted by water and/or carbon dioxide and/or ammonia) exist, with ammonia in excess over the stoichiometric amount being fed to the bottom of said column and carbon dioxide being fed to the central zone of said column, with a liquid bottom stream containing urea, ammonia and small amounts of water and a overhead gas and/or supercritical stream constituted by ammonia and water being obtained;
(b) feeding the liquid stream coming from the bottom of the reactive distillation column to one or more separation steps, with essentially containing ammonia streams, which are recycled to said column, and an urea containing stream, which is sent to the subsequent prilling step, being obtanined;
(c) feeding the gas and/or supercritical stream coming from the head of the reactive distillation column to a distillation column, from the bottom of which water is separated, whilst from the head thereof ammonia is discharged, which is directly recycled to said reactive distillation column.

The number of steps of separation of the liquid stream coming from the bottom of the reactive distillation column is so selected that a product is obtained which displays with a suitable purity level for being sent to the prilling step.

The gas and/or supercritical phase must be in a large enough amount to make it possibile most water formed during the carbamate transformation to be removed and/or to favour the decomposition of not converted carbamate into urea in the bottom portion of the column. Optionally, a portion of fresh and/or recycled ammonia can be fed to the central zone of the reactive distillation column together with carbon dioxide. Carbon dioxide can be fed to the reactive distillation column also as ammonium carbamate obtained in an auxiliary reactor (which must be suitably equipped with a system for developed heat removal) causing ammonia and carbon dioxide to react with each other.

The reactive distillation column operates at temperatures which are preferably comprised within the range of from 135 to 250°C, more preferably comprised within the range of from 170 to 200°C, under pressures preferably comprised within the range of from 50 to 250 atm, more preferably of from 70 to 200 atm, and with molar ratios of ammonia to carbon dioxide preferably comprised within the range of from 3 to 6, more preferably of from 3.5 to 5.

Such a reactive distillation column must be preferably provided with a number of stages, in its zone comprised within the feed inlet and the bottom, large enough in order to make it possible both carbamate to be dehydrated into urea, or same unreacted carbamate to be decomposed into ammonia and CO₂, and water to be extracted from the liquid phase, and with a stage number between the feed inlet and the column head, which is large enough in order to make it possible any CO₂ present in the gas and/or supercritical phase above the feed level to be completely transformed into carbamate.

Furthermore, said reactive distillation column can be provided with suitable heat exchangers in order to control the temperature level thereof and, in particular, remove heat from the central and/or upper portion, and supply heat to the bottom portion, of the column.

The distillation column to which the overhead stream effluent from the reactive distillation column is sent, operates under pressure values which are preferably comprised within the range of from 2 to 30 atm, more preferably of from 4 to 20 atm.

The reactive distillation column between the feed inlet and the column head can be of the type with trays (valve trays, perforated trays, bell type trays, and so forth), and of the packed type (either structured or not structured), whilst under the feed inlet level, it is of the only tray type (with bell, perforated, valve tray types, and so forth).

The heat developed on the feed tray and, more generally, the temperature level of the column, is controlled by means of suitable heat exchange system and/or by acting on the temperature level of CO₂ and/or NH₃ streams fed to the reactor column.

The operation of separation of urea from ammonia carried out on the bottom stream from the reactive distillation column may be carried out in four steps, in the following sequence:
-- by means of a first pressure decrease (inside a stripper) down to slightly lower values (for example, with a decrease of from 0.2 to 20 atm) relatively to the column-reactor operating pressure; the thus liberated NH₃ may be directly recycled to the column-reactor through an ejector using, as the driver fluid, fresh NH₃ and NH₃ coming from the subsequent separation steps;
-- by means of a second, fairly high, pressure decrease, so as to come to a pressure comprised within the range of from 15 to 25 atm; the evolved NH₃ is condensed and is then recycled by pumping;
-- by means of a third pressure lowering, so as to reach a pressure comprised within the range of from 3 to 6 atm; also in this case, the evolved NH₃ is recycled by pumping;
-- by means of an end pressure lowering, so as to reach an end pressure comprised within the range of from 0.1 to 0.5 atm.

The gas leaving the fourth step can be recycled by pumping, or it can be sent to a water absorption column, from the bottom of which an ammonia-water solution is obtained which is fed to the distillation column, to which the overhead stream from the reactive distillation column is sent.

Water used in the absorbtion column can be drawn from the bottom stream from the distillation column to which the overhead stream from the reactive distillation column was sent. The trays of column-reactor under the level of CO₂ feed inlet must display such a hold-up of liquid matter, as to enable the formed ammonium carbamate stream stay time to be comprised within the range of from a few minutes up to some ten minutes, and therefore enable said ammonium carbamate to be dehydrated in order to be converted into urea; the same trays must be provided in a large enough number as to ensure the liquid-gas equilibrium steps to be provided which are necessary in order to perform water extraction and/or CO₂ stripping from liquid urea phase by the ammonia gas and/or supercritical phase, thus making it possible the total conversione of CO₂ to be accomplished owing to the continuous shift of chemical equilibrium.

The steps above the feed inlet level (at a lower temperature than of those under the same level) are used in order to capture, as ammonium carbamate, any free CO₂ present in the gas and/or supercritical phase in equilibrium on the feed tray: this action is performed by the excess of NH₃ over the stoichiometric amount, which constitutes the major component in the same gas and/or supercritical phase.

The separation carried out in this step (b) is decidedly less burdensome, from the view point of operations and investment costs, than the traditional separation process, characterized by the simultaneous presence of CO₂ (or of not converted carbamate), and does not imply the need for using water in order to circulate the recycled streams, with consequent further considerable benefits on the reaction taking place inside the column-reactor. The recycle is preferably performed to the bottom of the column-reactor, together with any fresh NH₃ as necessary for the reaction.

Finally, we may observe that also the stream leaving the distillation column head, to which the overhead stream from the column-reactor is sent, by not containing CO₂, can be recycled to the same column-reactor under conditions of complete absence of water, thus making it possible the used NH₃ excess to be totally recovered without any water being required as solvent means for ammonium carbamate and/or hydrogencarbonate, thus enabling the column-reactor to be operated under considerably more favourable conditions than as according to the traditional processes.

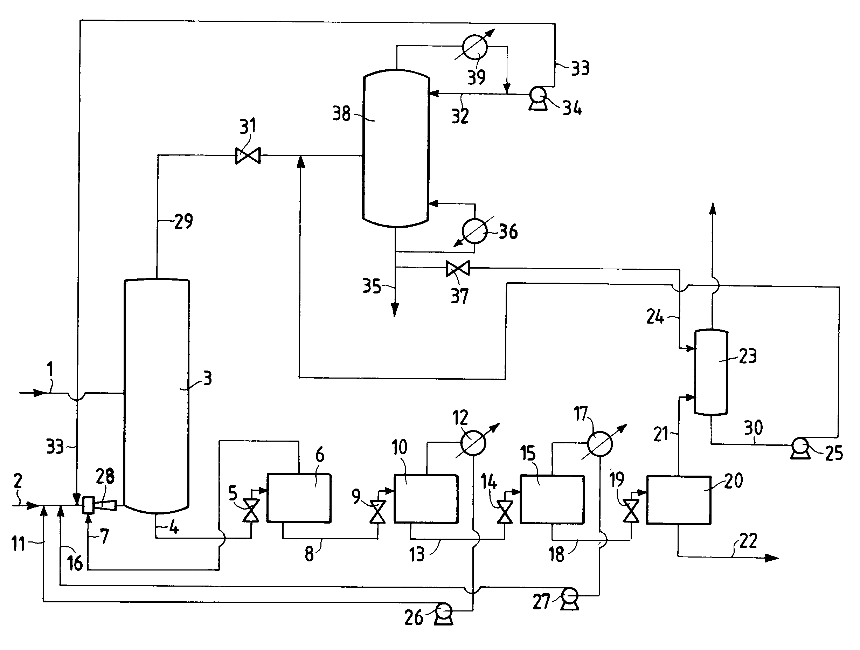

The present invention is better explained by referring to the schematic flow sheet of the accompanying figure, which displays a preferred embodiment not limitative of the same invention.

CO₂ is sent from line (1) to the column-reactor (3) provided with trays in its bottom portion and with packing material in the upper portion; fresh NH₃ is fed by means of the line (2) and through the ejector (28), after being combined with the recycled NH₃ streams (7), (11), (16), (33): the temperature on the feed tray is controlled by the temperature level of the feed streams and by an auxiliary heat exchanger (not shown).

The NH₃-urea mixture discharged by the bottom line (4) is depressurized in (5) and is expanded in (6); tha gas phase (7) is immediately recycled, and the liquid phase (8) is sent to (10) after being depressurized in (9) down to a medium-low pressure. The gas stream (11), substantially containing NH₃, is condensed in (12) and is recycled by means of the pump (26); the liquid phase (13), constituted by urea with a lower content of NH₃, is sent to the low-pressure depressurization step (15), downstream from the expansion valve (14). The gas stream (16), substantially containing NH₃, is condensed in (17) and is recycled by means of the pump (27), and the liquid phase (18) (molten urea with traces of NH₃) is sent to the end depressurization step (20), under vacuum, downstream from the expansion valve (19). The gas stream (21), substantially containing NH₃ discharged from (20), is stopped inside the vacuum absorber (23), and molten urea, completely free from NH₃, is sent to the end prilling step by means of the line (22); the water-NH₃ mixture discharged from the bottom of the absorber (23), line (30), is sent through the pump (25) to the rectification column (38) provided with the reboiler (36). The gas and/or supercritical stream (29), essentially constituted by NH₃ and water, discharged from the head of the column-reactor (3), is expanded in (31) and is sent to column (38), after being caused to flow through a heat exchanger (not shown). From the bottom of column (38) water free from NH₃ is discharged and is disposed of through the line (35), or is recycled to column (23) after depressurization in (37), to act as the solvent (24) in order to dissolve NH₃ coming from line (21). Ammonia leaving column (38) as the overhead stream thereof, after being condensed in (39), is refluxed from line (32) and is recycled to the feed line by means of the recycled line (33) and pump (34).

## Claims

1. Process for synthetizing urea from ammonia and carbon dioxide, with CO₂ being totally converted characterized in that said process comprises the following operations:
(a) feeding the reactants, in countercurrent mode, to a reactive distillation column (column-reactor) kept under such temperature and pressure conditions, that in each step a liquid phase and a gas and/or supercritical phase exist, with ammonia, used in excess over the stoichiometric amount, being to the bottom of said column and carbon dioxide being fed to the central zone of said column, with a liquid bottom stream containing urea, ammonia and small amounts of water and an overhead gas and/or supercritical stream constituted by ammonia and water being obtained;
(b) feeding the liquid stream coming from the bottom of the reactive distillation column to one or more separation steps, with essentially containing ammonia streams which are recycled to said column, and an urea containing stream which is sent to the subsequent prilling step,being obtained;
(c) feeding the gas and/or supercritical stream coming from the head of the reactive distillation column to a distillation column, from the bottom of which water is separated, whilst from the head thereof ammonia is discharged, which is directly recycled to said reactive distillation column.

2. Process according to claim 1, in which a portion of starting and/or recycled ammonia is fed to the central zone of the reactive distillation column together with carbon dioxide.

3. Process according to claim 2, in which carbon dioxide is fed to the reactive distillation column as ammonium carbamate obtained in an auxiliary reactor by starting from ammonia and carbon dioxide.

4. Process according to claim 1, or 2, or 3, in which the reactive distillation column operates at temperatures comprised within the range of from 135 to 250°C, pressures comprised within the range of from 50 to 250 atm, and with molar ratios of ammonia to carbon dioxide comprised within the range of from 3 to 6.

5. Process according to claim 4, in which the reactive distillation column operates at temperatures comprised within the range of from 170 to 200°C, pressures comprised within the range of from 70 to 200 atm and with molar ratios of ammonia to carbon dioxide comprised within the range of from 3.5 to 5.

6. Process according to claim 1, in which the reactive distillation column in the zone comprised between carbon dioxide feed level and the column bottom is a tray column.

7. Process according to claim 1, in which the reactive distillation column in the zone comprised between carbon dioxide feed level and column head is a tray column and/or a packed column.

8. Process according to claim 1, in which the distillation column to which the overhead stream from the reactive distillation column is sent, operates under pressures comprised within the range of from 2 to 30 atm.

9. Process according to claim 8, in which said pressure is comprised within the range of from 4 to 20 atm.

10. Process according to any of claims from 1 to 9, in which the separation of urea from ammonia, carried out on the bottom stream from the reactive distillation column is carried out in four steps, of which the first one is carried out at a slightly lower pressure than of the reactive distillation column, the second step is carried out at a pressure comprised within the range of from 15 to 25 atm, the third step is carried out at a pressure comprised within the range of from 3 to 6 atm, the fourth step is carried out at a pressure comprised within the range of from 0.1 to 0.5 atm.

11. Process according to claim 10, in which ammonia discharged from the last step of vacuum separation is sent to a water absorbtion column, from whose bottom an ammonia-water solution is obtained, which is sent to the distillation column to which the overhead stream from the reactive distillation column is sent.

12. Process according to claim 11, in which water used in the absorbtion column is drawn from the bottom stream from the distillation column to which the overhead stream from the reactive distillation column was sent.
